# EUROPEAN PATENT SPECIFICATION

(11) **EP 3 727 448 B1**
(45) Date of publication and mention of the grant of the patent: **14.07.2021**
(21) Application number: 18825693.7
(22) Date of filing: 20.12.2018
(51) Int. Cl.: A61K 41/00

(54) **METHOD FOR MANUFACTURING MESOPOROUS SILICA NANOPARTICLES WITH ENCAPSULATED SUPERPARAMAGNETIC CLUSTERS**
VERFAHREN ZUR HERSTELLUNG VON MESOPORÖSEN SILIZIUMDIOXID-NANOPARTIKELN MIT EINGEKAPSELTEN SUPERPARAMAGNETISCHEN CLUSTERN
PROCÉDÉ DE FABRICATION DE NANOPARTICULES DE SILICE MÉSOPOREUSE POURVUES DE D'AGRÉGATS SUPERPARAMAGNÉTIQUES ENCAPSULÉS

(30) Priority: 20.12.2017 LU 100581
(43) Date of publication of application: 28.10.2020
(73) Proprietor: Luxembourg Institute of Science and Technology (LIST), 4362 Esch-sur-Alzette (LU)
(72) Inventor: CORNE, Gaëlle, 54560 Audun-le-Roman (FR); THOMANN, Jean-Sébastien, 54730 Gorcy (FR)
(74) Representative: Lecomte & Partners
(86) International application number: PCT/EP2018/086189
(87) International publication number: WO 2019/122124

(56) References cited:
- WO-A1-2017/013250
- WO-A2-2008/140624
- CRUZ PAULA ET AL: "Titanium alkoxides immobilized on magnetic mesoporous silica nanoparticles and their characterization by solid state voltammetry techniques: Application in ring opening polymerization", MICROPOROUS AND MESOPOROUS MATERIALS, ELSEVIER, AMSTERDAM, NL, vol. 240, 28 November 2016 (2016-11-28), pages 227-235, XP029919254, ISSN: 1387-1811, DOI: 10.1016/J.MICROMESO.2016.11.028

## Description

### Technical field

The invention is directed to the field of mesoporous silica nanoparticles encapsulating superparamagnetic clusters. The method of manufacturing those nanoparticles is exemplified as well as their use in the release of active moieties, in particular fluorescent agents.

### Background art

Mesoporous silica nanoparticles are used in several biomedical applications, such as drug administration, therapeutic imaging and diagnostic.

When such nanoparticles are coated with a negatively charged supported lipid bilayer, it has been demonstrated that drug delivery is considerably enhanced. Hemocompatibility and biocompatibility in general are the key factors that have been put forward with this kind of coating.

On the other hand, magnetic hyperthermia, which is used in the treatment of cancer, uses magnetic nanoparticles to increase the tumor temperature once subjected to an external high-frequency electromagnetic field. In fact, the external high-frequency electromagnetic filed is used to trigger the drug delivery.

The loading of magnetic core within a mesoporous silica nanoparticles has already been achieved but it has been difficult to control the number of magnetic core within the nanoparticles.

The Stöber process (Stöber W. et al., J. Coll. Interf. Sci., 1968, 26, 62-69) concerns a system of chemical reactions that permits the controlled growth of spherical silica particles of uniform size by means of hydrolysis of alkyl silicates and subsequent condensation of silicic acid in alcoholic solution, using ammonia as a morphological catalyst.

Lu Y. et al. (Nano Lett., 2002, 2; 183-186) describe a sol-gel approach for the coating of superparamagnetic iron oxide nanoparticles with uniform shells of amorphous silica. The thickness of the silica coating has been controlled by changing the concentration of the sol-gel solution. Fluorescent dyes have been incorporated. However, a mixture of two types of iron oxide particles have been demonstrated: there is coexistence of maghemite and magnetite. Moreover, nanoparticles comprising only one magnetic core have been obtained.

Cruz P. et al. (Micro. Meso. Mat., 2017, 240, 227-235) have presented a synthesis of mesoporous silica coated Fe₃O₄ core-shell nanoparticles or magnetic mesoporous silica nanoparticles *via* a surfactant templated sol-gel method using magnetite nanoparticles stabilized with tartrate as nucleation seeds. 1 to 3 magnetic cores have been randomly encapsulated in one nanoparticle.

Egodawatte S. et al. (Micro. Meso. Mat., 2017, 237, 108-116) have loaded an anticancer drug into magnetic iron oxide/mesoporous silica nanocomposites with and without aminopropyl functionalization using three different solvents. They have used a surfactant template (hexadecyltrimethyl-ammonium bromide) to generate the micelles, which then trapped the iron oxide nanoparticles. Upon addition of tetraethoxysilane, the mesoporous silica shell is formed, resulting in several iron oxide nanoparticles inside a mesoporous silica shell. No control of the number of iron oxide nanoparticles is performed. As a result, the nanocomposites obtained formed a heterogeneous mixture. Some of the nanocomposites have an agglomerate of iron oxide nanoparticles within their shells, while others have only one or two nanoparticles.

All these disparities within the known nanocomposite lead to inhomogeneous release of active compounds (drugs, imaging reagents..) inside a living body.

### Summary of invention

### Technical Problem

The invention has for technical problem to alleviate at least one of the drawbacks present in the prior art. More particularly, the invention has for technical problem to provide a method of synthesis of silica nanoparticles with efficient encapsulation superparamagnetic cluster whose the number can be controlled.

### Technical solution

The first object of the invention is directed to a method for manufacturing mesoporous silica nanoparticles with encapsulated superparamagnetic clusters, said method comprising the following steps:
a. coating superparamagnetic nanoparticles with an hydrophobic layer,
b. dispersing said superparamagnetic nanoparticles in an organic apolar solvent,
c. evaporating said organic apolar solvent under vacuum, so as to obtain a wax constituted of superparamagnetic nanoparticles coated with an hydrophobic layer,
d. emulsifying said wax with a first cationic surfactant dispersed in an aqueous solution,
e. performing a first homogenisation of said solution,
f. adding a basic compound and a polar organic solvent in said solution,
g. performing a second homogenisation of said solution,
h. heating said solution to a temperature of at least 60°C,
i. adding a silicate precursor in said solution,
j. stirring said solution at a temperature of at least 60°C, and
k. performing a centrifugation in order to yield mesoporous silica nanoparticles with encapsulated superparamagnetic clusters.

Said method for manufacturing mesoporous silica nanoparticles with encapsulated superparamagnetic clusters is remarkable in that a second cationic surfactant is added between said steps (j) and (k).

According to a preferred embodiment, said superparamagnetic nanoparticles are superparamagnetic nanoparticles of metal oxide, preferentially of Fe₃O₄, CoFe₂O₄, MnFe₂O₄, or Zn_{0.4}Co_{0.6}Fe₂O₄.

According to a preferred embodiment, said hydrophobic layer is a layer of fatty acid, preferentially of oleic acid.

According to a preferred embodiment, said organic apolar solvent is selected from the group of pentane, hexane, cyclohexane, heptane or toluene.

According to a preferred embodiment, said first cationic surfactant and said second cationic surfactant are identical or different.

According to a preferred embodiment, said first cationic surfactant and/or said second cationic surfactant is a quaternary ammonium based surfactant, preferentially selected from the group comprising cetyltrimethylammonium chloride, cetrimonium bromide, or 1,2-dioleoyl-3-trimethylammonium-propane.

According to a preferred embodiment, said aqueous solution comprises milliQ water.

According to a preferred embodiment, said basic compound is triethanolamine, sodium hydroxide, or ammonia.

According to a preferred embodiment, said steps (e) and/or (g) are performed by ultrasonication and/or by stirring.

According to a preferred embodiment, said silicate precursor is ethoxysilane, methoxysilane, or tetraethyl orthosilicate, preferably tetraethyl orthosilicate.

According to a preferred embodiment, said temperature of said steps (h) and/or (j) is comprised between 60°C and 80°C.

According to a preferred embodiment, the number of equivalent of the total amount of said first and second cationic surfactant is comprised between 15 and 25 equivalents of said superparamagnetic nanoparticles, preferentially is equal to 18.

According to a preferred embodiment, the first cationic surfactant is comprised between 5% and 15% of the total amount of said first and second cationic surfactant, said first cationic surfactant being preferentially equivalent to 10% of the total amount of said first and second cationic surfactant.

According to a preferred embodiment, the first cationic surfactant is comprised between 16% and 22% of the total amount of said first and second cationic surfactant, said first cationic surfactant being preferentially equivalent to 19% of the total amount of said first and second cationic surfactant.

According to a preferred embodiment, the first cationic surfactant is comprised between 25% and 99% of the total amount of said first and second cationic surfactant, said first cationic surfactant being preferentially equivalent to 29% of the total amount of said first and second cationic surfactant.

The second object of the invention is directed to a method for manufacturing a negatively charged supported lipid bilayer surrounding a mesoporous silica nanoparticles with encapsulated superparamagnetic clusters, said method comprising the method in accordance with the first object of the invention and the steps of
a. adding (3-aminopropyl)triethoxysilane, alias APTES,
b. carrying out a purification,
c. adding a formulation of lipids, said lipids being 1,2-dioleoyl-sn-glycero-3-phospho-L-serine alias DOPS, cholesterol and at least one lipid different from DOPS and cholesterol,
d. performing an ultrasonication, and
e. performing a centrifugation.

According to a preferred embodiment, the number of equivalent of cholesterol relative to one equivalent of DOPS is comprised between 2.30 and 2.70, preferentially between 2.40 and 2.60, more preferentially the number of equivalent of cholesterol relative to one equivalent of DOPS is 2.50.

According to a preferred embodiment, said at least one lipid different from DOPS and cholesterol is 1,2-dipalmitoyl-sn-glycero-3-phosphocholine alias DPPC.

The third object of the invention is directed to a method for manufacturing a layer of alginate on negatively charged supported lipid bilayer surrounding a mesoporous silica nanoparticles with encapsulated superparamagnetic clusters, said method comprising the method for manufacturing a negatively charged supported lipid bilayer surrounding a mesoporous silica nanoparticles with encapsulated superparamagnetic clusters in accordance with the second object of the invention, and the steps of
a. providing a negatively charged supported lipid bilayer surrounding a mesoporous silica nanoparticle with encapsulated superparamagnetic clusters, wherein said negatively charged supported lipid bilayer comprises at least one phospholipid,
b. adding a formulation of lipid, said lipids being preferentially 1,2-dioleoyl-3-trimethylammonium-propane alias DOTAP, cholesterol and at least one lipid different from DOTAP and cholesterol,
c. performing an ultra-sonication,
d. adding an aqueous solution of sodium alginate, and
e. cross-linking said sodium alginate.

According to a preferred embodiment, said at least one lipid different from DOTAP and cholesterol is 1,2-dipalmitoyl-sn-glycero-3-phosphocholine alias DPPC.

The fourth object of the invention is directed to mesoporous silica nanoparticle comprising at least one encapsulated superparamagnetic cluster. Said mesoporous silica nanoparticle is remarkable in that said at least one encapsulated superparamagnetic cluster is centred inside said mesoporous silica nanoparticles.

According to a preferred embodiment, said mesoporous silica nanoparticle comprises three or five encapsulated superparamagnetic clusters.

According to a preferred embodiment, said encapsulated superparamagnetic clusters are aligned.

According to a preferred embodiment, said encapsulated superparamagnetic clusters are superparamagnetic clusters of metal oxide, preferentially of Fe₃O₄, CoFe₂O₄, MnFe₂O₄, or Zn_{0.4}Co_{0.6}Fe₂O₄.

The fifth object of the present invention is directed to mesoporous silica nanoparticle with a negatively charged supported lipid bilayer, said mesoporous silica nanoparticle comprising at least one encapsulated superparamagnetic cluster. Said mesoporous silica nanoparticle is remarkable in that said at least one encapsulated superparamagnetic clusters is encapsulated metal oxide, preferentially superparamagnetic nanoparticles of Fe₃O₄, CoFe₂O₄, MnFe₂O₄, or Zn_{0.4}Co_{0.6}Fe₂O₄.

According to a preferred embodiment, said nanoparticle comprises one, three or five encapsulated superparamagnetic clusters.

According to a preferred embodiment, said negatively charged supported lipid bilayer comprises cholesterol, 1,2-dioleoyl-sn-glycero-3-phospho-L-serine alias DOPS, and at least one lipid different from DOPS and cholesterol.

According to a preferred embodiment, said at least one lipid different from DOPS and cholesterol is 1,2-dipalmitoyl-sn-glycero-3-phosphocholine alias DPPC.

According to a preferred embodiment, said mesoporous silica nanoparticle with a negatively charged supported lipid bilayer in accordance with the fifth object of the invention further comprises an outer layer of alginate.

The sixth object of the invention is directed to mesoporous silica nanoparticles with a negatively charged supported lipid bilayer in accordance with the fifth objection of the invention for use in magnetic hyperthermia treatment, or in post-treatment of cancerous tumour.

The seventh object of the invention is directed to mesoporous silica nanoparticles with a negatively charged supported lipid bilayer in accordance with the fifth object of the invention further comprising an outer layer of alginate for use in loco-regional and/or topical administration as a cosmetic agent and/or a dermatological agent.

The eighth object of the invention is directed to mesoporous silica nanoparticle with a negatively charged supported lipid bilayer in accordance with the fourth object of the invention, the fifth object of the invention and/or the fifth object of the invention further comprising an outer layer of alginate for use in magnetic resonance imaging.

The ninth object of the invention is directed to mesoporous silica nanoparticle with a negatively charged supported lipid bilayer in accordance with the fourth object of the invention, fifth object of the invention and/or the fifth object of the invention further comprising an outer layer of alginate for simultaneous use in magnetic resonance imaging and fluorescence imaging.

### Advantages of the invention

The invention is particularly interesting in that the method for manufacturing the mesoporous silica nanoparticles with the encapsulated magnetic clusters allows for the control of the number of cores of metal oxide within the silica porous matrix.

Such cores are also centred into the mesoporous silica nanoparticles, allowing for enhanced properties in magnetic hyperthermia treatment.

The superparamagnetic cores or clusters, when present in a number superior to 1, are also aligned inside the mesoporous silica nanoparticle. This confers a shape anisotropy which increases the heating efficiency in the use of the nanoparticles of the present invention in magnetic hyperthermia treatment.

The method of the present invention is a one-pot method, not requiring any purification steps.

The yield of the mesoporous silica nanoparticles is significantly high, since the control of the number of magnetic cores within the silica nanoparticles allows for obtaining homogeneous nanoparticles. Every nanoparticle has its own magnetic cores.

### Brief description of the drawings

Figure 1: Scanning Electron Microscopy (SEM) image of nanoparticles of Fe₃O₄ covered with oleic acid.
Figure 2: Scanning Electron Microscopy (SEM) images of the synthesis of the mesoporous silica nanoparticles with encapsulated superparamagnetic clusters in accordance with the amount of cationic surfactant.
Figure 3: Vial with a mesoporous silica nanoparticle with a supported lipid bilayer with rhodamine B incorporated and without encapsulated superparamagnetic clusters (on the left) and vial with a mesoporous silica nanoparticle with a supported lipid bilayer with rhodamine B incorporated and with encapsulated superparamagnetic clusters (on the right) before centrifugation.
Figure 4: Vial with a mesoporous silica nanoparticle with a supported lipid bilayer with rhodamine B incorporated and without encapsulated superparamagnetic clusters (on the left) and vial with a mesoporous silica nanoparticle with a supported lipid bilayer with rhodamine B incorporated and with encapsulated superparamagnetic clusters (on the right) after centrifugation.

### Description of an embodiment

The synthesis of the mesoporous silica nanoparticles with a controlled amount of encapsulated superparamagnetic cluster is performed in two main steps. The first main step concerns the nucleation of the superparamagnetic particles and the second main steps concerns the condensation of silica in order to wrap those superparamagnetic particles.

The nucleation of the superparamagnetic particles (namely, the first main step of the synthesis) is conducted as following:

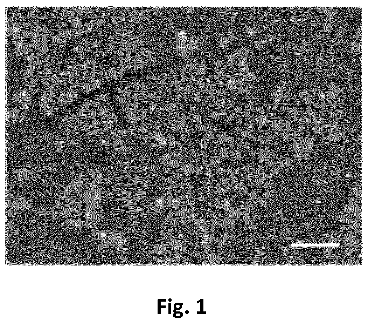

The superparamagnetic particles of metal oxide are first coated with a hydrophobic layer. The hydrophobic layer can be a layer of fatty acid, for example oleic acid. Figure 1 shows an image obtained by scanning electron microscopy (SEM) of superparamagnetic particles covered with oleic acid. It forms nanoemulsions that cannot be subsequently dispersed into water. However, it can be and it is dispersed into an organic apolar solvent, which can be for instance hexane. Pentane, heptane, cyclohexane or toluene can also be used.

The superparamagnetic particles of metal oxide are advantageously superparamagnetic particles of iron (II, III) oxide (Fe₃O₄), CoFe₂O₄, MnFe₂O₄, or Zn_{0.4}Co_{0.6}Fe₂O₄, more preferentially Fe₃O₄.

Once dispersed, the organic apolar solvent is evaporated under vacuum in order to add the dispersed superparamagnetic particles into an aqueous solution, preferentially comprising milliQ water and a first cationic surfactant.

Then, by emulsifying the obtained wax, a fatty nanoemulsion is obtained. This fatty phase (due to the presence of oleic acid) is in fact stabilized by the cationic surfactant.

The first cationic surfactant is advantageously cetyltrimethylammonium chloride (CTACI). Cetrimonium bromide (CTAB), 1,2-dioleoyl-3-trimethylammonium-propane (DOTAP) or any other quaternary ammonium-based surfactant can be employed.

According to the amount of cationic surfactant present into the solution, it is possible to control the number of magnetic cores/clusters that will be present at the end of the synthesis, within the mesoporous silica nanoparticles.

The condensation of silica (namely, the second main steps of the synthesis) is then conducted as following:

A basic compound and a polar organic solvent are added in the homogenized mixture.

The basic compound is advantageously triethanolamine. Triethylamine, ammonia or sodium hydroxide can also been employed.

The polar organic solvent is advantageously ethanol. Methanol or isopropanol can also been employed.

The solution that is thus obtained is then stirred and heated to at least 60°C. A temperature comprised between 60°C and 80°C is generally set up.

The silica precursor is added at this temperature.

The silica precursor is advantageously tetraethyl orthosilicate, ethoxysilane, methoxysilane. Any other alkoxysilanes, halogenosilane, silatranes, oligomers or monomers of alkoxysilanes, of halogenosilanes or of silatranes can be used.

A second cationic surfactant is then added to stabilize the fatty phase is added. It acts as a porogen agent that is helpful for the growth of the mesoporous silica shell.

The second cationic surfactant can be identical or different to the first cationic surfactant. The second cationic surfactant is advantageously cetyltrimethylammonium chloride (CTACI). Cetrimonium bromide (CTAB), 1,2-dioleoyl-3-trimethylammonium-propane (DOTAP) or any other quaternary ammonium-based surfactant can be employed.

The solution is stirred at a temperature of at least 60°C, in order to carry out the condensation of the silica around the super-paramagnetic core. A temperature comprised between 60°C and 80°C is generally set up.

Finally, after a centrifugation step, the mesoporous silica nanoparticles are obtained.

The main idea of this above described process is that the total amount of the first and the second cationic surfactant is added in two parts: a first portion is required to emulsify the fatty phase (wax) and a second portion is required to participate to the condensation of the silica precursors; in this case the first and second cationic surfactant are identical. In the case where the first and second surfactant are different, the first surfactant is required to emulsify the fatty phase (wax) and the second surfactant (different from the first one) is required to participate to the condensation of the silica precursors. There is a preference for the second surfactant to be cetyltrimethylammonium chloride (CTACI) since it performs better in the condensation of the silica precursors.

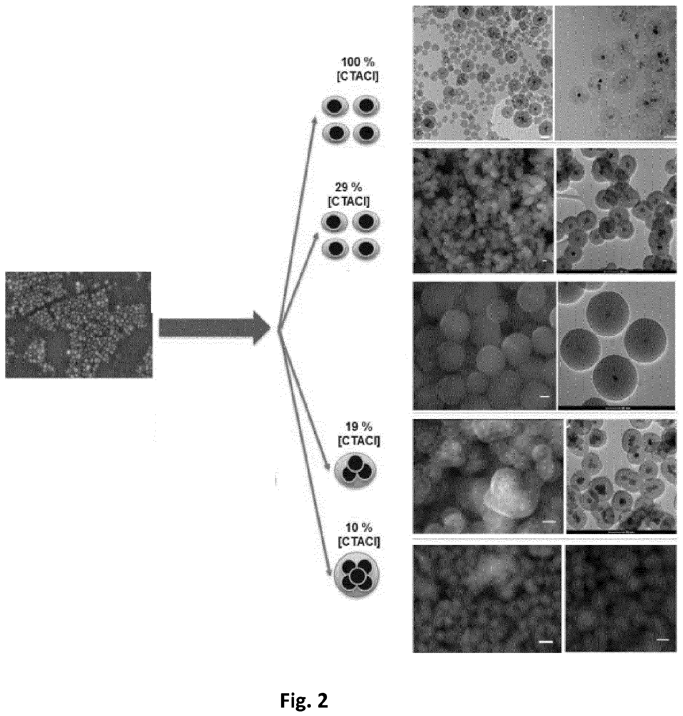

In the following, the total amount of cationic surfactant with regard to the number of magnetic cores/clusters encapsulated in the final nanoparticles is indicated. This is shown in figure 2, which further depicts SEM images. In this case (shown on figure 2), the first and second cationic surfactant are therefore identical, cetyltrimethylammonium chloride (CTACI) having been used.

For 100 mg of iron (II, III) oxide (Fe₃O₄) nanoparticles covered with oleic acid, the total amount of cetyltrimethylammonium chloride is 10.4 mL (7.8 mmol). That means that the number of equivalent of CTACI compared to the one equivalent of metal oxide is equal to 18. Generally speaking the total number of equivalent of the cationic surfactant must be comprised between 15 and 25.

When the first portion of cetyltrimethylammonium chloride amounts to 10% of the total amount, then five superparamagnetic cores are included in the mesoporous silica shell. This result can be also obtained when the amount is comprised between 5% and 15% of the total amount of CTACI.

When the first portion of cetyltrimethylammonium chloride amounts to 19% of the total amount, then three superparamagnetic cores are included in the mesoporous silica shell. This result can be also obtained when the amount is comprised between 16% and 22% of the total amount of CTACI.

When the first portion of cetyltrimethylammonium chloride is above 25% (for instance 29% or in the case where the total amount is added in the first portion) of the total amount, then only one superparamagnetic core is included in the mesoporous silica shell.

When the number of superparamagnetic clusters is greater than 1, for example three or five as in the above examples, it has been observed that the clusters are centred within the mesoporous silica nanoparticles. There are also aligned, forming thus a nanochain of superparamagnetic clusters. It has been demonstrated that chain-like structures can give rise to an increase in the heating efficiency of the nanoparticles, notably in the magnetic hyperthermia treatment of cancers. This is due to uniaxial anisotropy phenomenon, which leads to hysteretic losses that can improve the heat power dissipation process (Abenojar E. C. et al., Prog. Nat. Sci. Mater. Intern., 2016, 26, 440-448). The development of a method of manufacturing in controlled and regulated manner those mesoporous silica nanoparticles with encapsulated superparamagnetic clusters is therefore significantly valuable in the field of magnetic hyperthermia.

Different implementations of the mesoporous silica nanoparticles with encapsulated superparamagnetic clusters have been developed.

For instance, in order to increase the biocompatibility of the mesoporous silica nanoparticles, negatively charged supported lipid bilayer surrounding the nanoparticles have been developed. The goal is to facilitate the incorporation of those nanoparticles in a living system and to use them as a nanovector for the encapsulation and the delivery of (active) materials.

Superparamagnetic nanoparticles of iron oxide have been covered with an hydrophobic layer of oleic acid. After dispersion in hexane, a wax has been generated by evaporating the solvent. The addition of the first cationic surfactant (CATCI) allows for the control of the number of superparamagnetic clusters in the nanoparticules. After homogeneisation by ultrasonication and addition of triethanolamine, the mixture has been heated up to at least 60°C. Tetraethyl orthosilicate has been added followed by the second cationic surfactant which is in this case identical to the first cationic surfactant.

In order to add the negatively charged supported lipid bilayer, the mesoporous silica nanoparticles must be rendered cationic. This is done by adding a layer of (3-aminopropyl)triethoxysilane (APTES).

After 20 minutes of reaction, 150 µL of APTES (0.64 mmol) are added to the reaction volume. The reaction mixture is stirred for 2 hours at 60 °C under argon. The molar ratio of this synthesis can be for example the following: TEOS / CTACI / TEA / H₂O / EtOH / APTES : 1 / 0.26 / 2 / 117.35/5.88/0.10.

Then a purification step is carried out, in order to remove the CTACI.

Then, a formulation of lipids is added. This formulation is composed of DOPS, cholesterol and a third lipid, preferentially DPPC.

The number of equivalents of cholesterol relative to one equivalent of DOPS is comprised between 2.35 and 2.65, preferentially between 2.40 and 2.60, more preferentially between 2.45 and 2.55.

The formulation of lipids has been prepared in a solvent that has been evaporated before mixing with the mesoporous silica nanoparticles.

An ultra-sonication step, preferentially carried out at room temperature, under argon, and during 20 minutes, is carried out.

After a centrifugation, the mesoporous silica nanoparticles with encapsulated superparamagnetic clusters and with a negatively charged supported lipid bilayer are obtained.

This kind of mesoporous silica nanoparticles with encapsulated superparamagnetic clusters and with a negatively charged supported lipid bilayer are employed in magnetic hyperthermia treatment. There are also employed in post-treatment of cancerous tumour.

In order to enhance the biocompatibility of the nanoparticle, namely to provide a biocompatible nanocapsule with an outer layer that is sufficiently resistant to prevent leakage of the materials (and/or any active moieties), and to be sufficiently biodegradable at the same time to favour the slow and control release of said materials, an outer alginate layer is further provide to the mesoporous silica nanoparticles with encapsulated superparamagnetic clusters of the present invention. The outer alginate layer is designed over the negatively charged supported lipid bilayer.

To adsorb sodium alginate, liposomes comprising 1,2-dioleoyl-3-trimethylammonium-propane (DOTAP), cholesterol and a third lipid (preferentially DPPC) are added on the nanoparticles bearing the negatively charged supported lipid bilayer.

The concentration of DOTAP in the formulation is comprised between 50% and 150% of the concentration of said at least one phospholipid, preferentially in a concentration amounting to 100% of the concentration of said at least one phospholipid.

After ultra-sonication, preferentially performed at a temperature comprised between 40°C and 60°C, the suspension is then mixed under mechanical stirring with 2% by weight of sodium alginate (aqueous solution).

The concentration of sodium alginate in said aqueous solution is advantageously comprised between 1% (wt%) and 5% (wt%), preferentially is equal to 2% (wt%).

The mixing with sodium alginate is performed in a buffer solution, said buffer solution being based on 4-(2-hydroxyethyl)-1 piperazineethanesulfonic acid, alias HEPES, or on phosphate-buffered saline, alias PBS.

The cross-linking sodium alginate is performed by mixing an aqueous solution of divalent cation, preferentially calcium-based cation or barium-based cation, at a concentration comprised between 40 mM and 60 mM, preferentially at a concentration of 50 mM.

The excess of un-adsorbed alginate can be removed by centrifugation at 45000 rpm for 20 min.

The implementation in hyperthermia magnetic treatment has been carried out.

To quantify the encapsulation of active agents and their release, fluorescent agents have been used such as rhodamine B. For the adsorption of rhodamine B in the matrix of silica, 1 mg of rhodamine B is incorporated directly into the aqueous solution of silica (2 mg silica nanoparticles). Rhodamine B was incorporated into the silica matrix by adsorption in milliQ water by incubating overnight. The maximum absorbance of rhodamine B is 556 nm.

Calcein is another fluorescent dye that can be incorporated into the nanoparticles with superparamagnetic clusters.

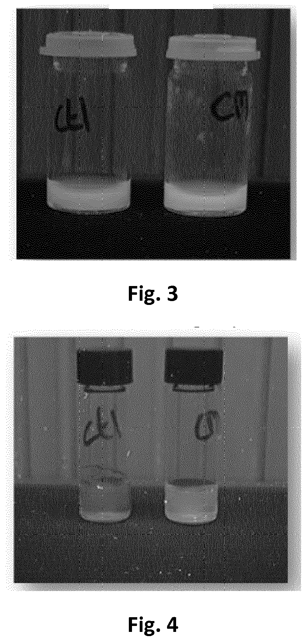

An alternating magnetic field with a frequency of 28.4 kHz and a radial magnetic field amplitude of 3010 A/m for a duration of two hours is provided onto said nanoparticles with superparamagnetic clusters and with rhodamine B. Once the magnetic field has been provided, an increase of fluorescence is observed after centrifugation, demonstrating the release of the fluorescent dye from the mesoporous silica nanoparticles (see figure 4, right vial). In fact, the heat provided by the excitation of the superparamagnetic cluster increases the fluidity of the supported lipid bilayer and provokes the release of the rhodamine B.

In comparison with mesoporous silica nanoparticles without superparamagnetic cluster, after centrifugation, no fluorescence has been observed (as in figure 3, left and right vials, where no centrifugation has been performed).

The centrifugation allows for the rhodamine B to be dispersed into the solution.

The mesoporous silica nanoparticles with a negatively charged supported lipid bilayer have found a use in magnetic hyperthermia treatment, or in post-treatment of cancerous tumour. Special attention has been provided to the magnetic hyperthermia treatment of glioblastoma.

The mesoporous silica nanoparticles with a negatively charged supported lipid bilayer and an outer layer of alginate have found a use in loco-regional and/or topical administration as a cosmetic agent and/or a dermatological agent.

The implementations of such mesoporous silica nanoparticles with the protective outer layer of cross-linked sodium alginate are various. It forms indeed nanocapsules in which several kinds of compositions can be incorporated. Compositions comprising contrasting agents, therapeutic molecules, pharmaceutical agents, cosmetic agents, dermatological agents (wound-healing agents), anticancerous agents, nutraceutical agents, cosmeceutical agents, hormonal products, growth factors and/or compounds adapted for loco-regional therapies (bone implants and/or cardiac implants), for intraocular administration and/or for intratumoral agent, can be thus easily derivatized.

Moreover, applications of the mesoporous silica nanoparticle comprising encapsulated superparamagnetic cluster in magnetic resonance imaging (MRI) and in bimodal imaging (which is a combination of MRI and fluorescence imaging) are envisioned, especially since the number of superparamagnetic clusters can be controlled by the method of the present invention. The major advantage is to be able to perform a (medical) treatment while performing a (medical) imaging at the same time. The release of the drug or the active moiety is indeed achieved while imaging. According to the specific application, those nanoparticles comprising encapsulated superparamagnetic clusters are used with or without negatively charged supported lipid bilayer. When the bilayer is used, an outer layer of alginate can still enhance the biocompatibility of those nanocapsules.

## Claims

1. Method for manufacturing mesoporous silica nanoparticles with encapsulated superparamagnetic clusters, said method comprising the following steps:
a) coating superparamagnetic nanoparticles with an hydrophobic layer,
b) dispersing said superparamagnetic nanoparticles in an organic apolar solvent,
c) evaporating said organic apolar solvent under vacuum, so as to obtain a wax constituted of superparamagnetic nanoparticles coated with an hydrophobic layer,
d) emulsifying said wax with a first cationic surfactant dispersed in an aqueous solution,
e) performing a first homogenisation of said solution,
f) adding a basic compound and a polar organic solvent in said solution,
g) performing a second homogenisation of said solution,
h) heating said solution to a temperature of at least 60°C,
i) adding a silicate precursor in said solution,
j) stirring said solution at a temperature of at least 60°C, and
k) performing a centrifugation in order to yield mesoporous silica nanoparticles with encapsulated superparamagnetic clusters;
**characterized in that**
a second cationic surfactant is added between said steps (j) and (k).

2. Method according to claim 1, **characterized in that** said superparamagnetic nanoparticles are superparamagnetic nanoparticles of metal oxide, preferentially of Fe₃O₄, CoFe₂O₄, MnFe₂O₄, or Zn_{0.4}Co_{0.6}Fe₂O₄.

3. Method according to claim 1 or 2, **characterized in that** said first cationic surfactant and/or said second cationic surfactant is a quaternary ammonium based surfactant, preferentially selected from the group comprising cetyltrimethylammonium chloride, cetrimonium bromide, or 1,2-dioleoyl-3-trimethylammonium-propane.

4. Method according to any one of claims 1-3, **characterized in that** the number of equivalent of the total amount of said first and second cationic surfactant is comprised between 15 and 25 equivalents of said superparamagnetic nanoparticles, preferentially is equal to 18.

5. Method according to any one of claims 1-4, **characterized in that** the first cationic surfactant is comprised between 5% and 15% of the total amount of said first and second cationic surfactant, said first cationic surfactant being preferentially equivalent to 10% of the total amount of said first and second cationic surfactant.

6. Method for manufacturing a negatively charged supported lipid bilayer surrounding a mesoporous silica nanoparticles with encapsulated superparamagnetic clusters, said method comprising the method for manufacturing mesoporous silica nanoparticles with encapsulated superparamagnetic clusters in accordance with any one of claims 1-5 and the steps of
a) adding (3-aminopropyl)triethoxysilane, alias APTES,
b) carrying out a purification,
c) adding a formulation of lipids, said lipids being 1,2-dioleoyl-sn-glycero-3-phospho-L-serine alias DOPS, cholesterol and at least one lipid different from DOPS and cholesterol,
d) performing an ultrasonication, and
e) performing a centrifugation.

7. Method according to claim 6, wherein the number of equivalent of cholesterol relative to one equivalent of DOPS is comprised between 2.30 and 2.70, preferentially between 2.40 and 2.60, more preferentially the number of equivalent of cholesterol relative to one equivalent of DOPS is 2.50.

8. Method for manufacturing a layer of alginate on negatively charged supported lipid bilayer surrounding a mesoporous silica nanoparticles with encapsulated superparamagnetic clusters, said method comprising the method for manufacturing a negatively charged supported lipid bilayer surrounding a mesoporous silica nanoparticles with encapsulated superparamagnetic clusters in accordance with any one of claims 6-7, and the steps of
a) providing a negatively charged supported lipid bilayer surrounding a mesoporous silica nanoparticle with encapsulated superparamagnetic clusters, wherein said negatively charged supported lipid bilayer comprises at least one phospholipid,
b) adding a formulation of lipid, said lipids being preferentially 1,2-dioleoyl-3-trimethylammonium-propane alias DOTAP, cholesterol and at least one lipid different from DOTAP and cholesterol,
c) performing an ultra-sonication,
d) adding an aqueous solution of sodium alginate, and
e) cross-linking said sodium alginate.

9. Method according to claim 8, **characterized in that** said at least one lipid different from DOTAP and cholesterol is 1,2-dipalmitoyl-sn-glycero-3-phosphocholine alias DPPC.

10. Mesoporous silica nanoparticle comprising at least one encapsulated superparamagnetic cluster, **characterized in that** said at least one encapsulated superparamagnetic cluster is centred inside said mesoporous silica nanoparticle, and said mesoporous silica nanoparticle comprising three or five aligned encapsulated superparamagnetic clusters.

11. Mesoporous silica nanoparticle according to claim 10, **characterized in that** said encapsulated superparamagnetic cluster are one superparamagnetic cluster of metal oxide, preferentially of Fe₃O₄, CoFe₂O₄, MnFe₂O₄, or Zn_{0.4}Co_{0.6}Fe₂O₄.

12. Mesoporous silica nanoparticle according claim 10 or 11, **characterized in that** said mesoporous nanoparticle comprises a negatively charged supported lipid bilayer and further comprising an outer layer of alginate.

13. Mesoporous silica nanoparticle according to claim 12, for use in magnetic hyperthermia treatment or in post-treatment of cancerous tumour.

14. Mesoporous silica nanoparticle in accordance with claim 12, for use in loco-regional and/or topical administration as a cosmetic agent and/or a dermatological agent.

15. Mesoporous silica nanoparticle in accordance with any one of claims 10-14, for use in magnetic resonance imaging or for simultaneous use in magnetic resonance imaging and fluorescence imaging.

## Patentansprüche

1. Verfahren zum Herstellen von mesoporösen Siliciumdioxid-Nanopartikeln mit verkapselten superparamagnetischen Clustern, wobei das Verfahren die folgenden Schritte umfasst:
a) Beschichten von superparamagnetischen Nanopartikeln mit einer hydrophoben Schicht,
b) Dispergieren der superparamagnetischen Nanopartikel in einem organischen apolaren Lösungsmittel,
c) Verdampfen des organischen apolaren Lösungsmittels unter Vakuum, um ein Wachs zu erhalten, das von superparamagnetischen Nanopartikeln gebildet wird, die mit einer hydrophoben Schicht überzogen sind,
d) Emulgieren des Wachses mit einem ersten kationischen Tensid, das in einer wässrigen Lösung dispergiert ist,
e) Durchführen einer ersten Homogenisierung der Lösung,
f) Hinzufügen einer basischen Verbindung und eines polaren organischen Lösungsmittels zu der Lösung,
g) Durchführen einer zweiten Homogenisierung der Lösung,
h) Erwärmen der Lösung auf eine Temperatur von mindestens 60 °C,
i) Hinzufügen einer Silikatvorstufe zu der Lösung,
j) Rühren der Lösung bei einer von Temperatur von mindestens 60 °C, und
k) Durchführen einer Zentrifugierung, um mesoporöse Siliciumdioxid-Nanopartikel mit verkapselten superparamagnetischen Clustern zu erhalten;
**dadurch gekennzeichnet, dass**
zwischen den Schritten (j) und (k) ein zweites kationisches Tensid hinzugefügt wird.

2. Verfahren nach Anspruch 1, **dadurch gekennzeichnet, dass** die superparamagnetischen Nanopartikel superparamagnetische Nanopartikel aus Metalloxid sind, vorzugsweise aus Fe₃O₄, CoFe₂O₄, MnFe₂O₄ oder Zn_{0,4}Co_{0,6}Fe₂CO₄.

3. Verfahren nach Anspruch 1 oder 2, **dadurch gekennzeichnet, dass** das erste kationische Tensid und/oder das zweite kationische Tensid ein Tensid auf Basis einer quartären Ammoniumverbindung ist, vorzugsweise ausgewählt aus der Gruppe bestehend aus Cetyltrimethylammoniumchlorid, Cetrimoniumbromid oder 1,2-Dioleoyl-3-trimethylammoniumpropan.

4. Verfahren nach einem der Ansprüche 1-3, **dadurch gekennzeichnet, dass** die Zahl von Äquivalenten in der Gesamtmenge aus dem ersten und dem zweiten kationischen Tensid zwischen 15 und 25 Äquivalenten der superparamagnetischen Nanopartikel liegt, vorzugsweise gleich 18 ist.

5. Verfahren nach einem der Ansprüche 1-4, **dadurch gekennzeichnet, dass** das erste kationische Tensid zwischen 5 % und 15 % der Gesamtmenge aus dem ersten und dem zweiten kationischen Tensid ausmacht, wobei das erste kationische Tensid vorzugsweise 10 % der Gesamtmenge aus dem ersten und dem zweiten kationischen Tensid entspricht.

6. Verfahren zum Herstellen einer negativ geladenen, geträgerten Lipiddoppelschicht, die ein mesoporöses Siliciumdioxid-Nanopartikel mit verkapselten superparamagnetischen Clustern umgibt, wobei das Verfahren das Verfahren zum Herstellen mesoporöser Siliciumdioxid-Nanopartikel mit verkapselten superparamagnetischen Clustern gemäß einem der Ansprüche 1-5 und die folgenden Schritte umfasst:
a) Hinzufügen von (3-Aminopropyl)triethoxysilan alias APTES,
b) Ausführen einer Reinigung,
c) Hinzufügen einer Formulierung aus Lipiden, wobei es sich bei den Lipiden um 1,2-Dioleoyl-sn-glycero-3-phospho-L-serin alias DOPS, Cholesterol und mindestens ein von DOPS und Cholesterol verschiedenes Lipid handelt,
d) Durchführen einer Ultraschallbehandlung und
e) Durchführen einer Zentrifugierung.

7. Verfahren nach Anspruch 6, wobei die Zahl der Äquivalente von Cholesterol relativ zu einem Äquivalent von DOPS zwischen 2,30 und 2,70, vorzugsweise zwischen 2,40 und 2,60 liegt, wobei die Zahl der Äquivalente von Cholesterol relativ zu einem Äquivalent von DOPS stärker bevorzugt 2,50 ist.

8. Verfahren zum Herstellen einer Schicht aus Alginat auf einer negativ geladenen, geträgerten Lipiddoppelschicht, die ein mesoporöses Siliciumdioxid-Nanopartikel mit verkapselten superparamagnetischen Clustern umgibt, wobei das Verfahren das Verfahren zum Herstellen einer negativ geladenen, geträgerten Lipiddoppelschicht, die ein mesoporöses Siliciumdioxid-Nanopartikel mit verkapselten superparamagnetischen Clustern umgibt, gemäß einem der Ansprüche 6-7 und die folgenden Schritte umfasst:
a) Bereitstellen einer negativ geladenen, geträgerten Lipiddoppelschicht, die ein mesoporöses Siliciumdioxid-Nanopartikel mit verkapselten superparamagnetischen Clustern umgibt, wobei die negativ geladene, geträgerte Lipiddoppelschicht mindestens ein Phospholipid umfasst,
b) Hinzufügen einer Lipidformulierung, wobei es sich bei den Lipiden vorzugsweise um 1,2-Dioleoyl-3-trimethylammoniumpropan alias DOTAP, Cholesterol und mindestens ein von DOTAP und Cholesterol verschiedenes Lipid handelt,
c) Durchführen einer Ultraschallbehandlung,
d) Hinzufügen einer wässrigen Lösung aus Natriumalginat und
e) Vernetzen des Natriumalginats.

9. Verfahren nach Anspruch 8, **dadurch gekennzeichnet, dass** das mindestens eine Lipid, das von DOTAP und Cholesterol verschieden ist, 1,2-Dipalmitoyl-sn-glycero-3-phosphocholin alias DPPC ist.

10. Mesoporöses Siliciumdioxid-Nanopartikel, mindestens einen verkapselten superparamagnetischen Cluster umfassend, **dadurch gekennzeichnet, dass** der mindestens eine verkapselte superparamagnetische Cluster innerhalb des mesoporösen Siliciumdioxid-Nanopartikels zentriert ist und das mesoporöse Siliciumdioxid-Nanopartikel drei oder fünf auf einer Linie liegende verkapselte superparamagnetische Cluster umfasst.

11. Mesoporöses Siliciumdioxid-Nanopartikel nach Anspruch 10, **dadurch gekennzeichnet, dass** es sich bei dem verkapselten superparamagnetischen Cluster um einen superparamagnetischen Cluster aus Metalloxid, vorzugsweise aus Fe₃O₄, CoFe₂O₄, MnFe₂O₄ oder Zn_{0,4}Co_{0,6}Fe₂CO₄ handelt.

12. Mesoporöses Siliciumdioxid-Nanopartikel nach Anspruch 10 oder 11, **dadurch gekennzeichnet, dass** das mesoporöse Nanopartikel eine negativ geladene, geträgerte Lipiddoppelschicht umfasst und ferner eine Außenschicht aus Alginat umfasst.

13. Mesoporöses Siliciumdioxid-Nanopartikel nach Anspruch 12 zur Verwendung bei einer magnetischen Hyperthermiebehandlung oder bei einer Nachbehandlung eines krebsartigen Tumors.

14. Mesoporöses Siliciumdioxid-Nanopartikel nach Anspruch 12 zur Verwendung einer loko-regionalen und/oder topischen Anwendung als kosmetischer Wirkstoff und/oder als dermatologischer Wirkstoff.

15. Mesoporöses Siliciumdioxid-Nanopartikel nach einem der Ansprüche 10-14 zur Verwendung bei einer Kernspintomographie oder zur simultanen Verwendung bei einer Kernspintomographie und einer Fluoreszenzbildgebung.

## Revendications

1. Procédé de fabrication de nanoparticules de silice mésoporeuses avec des agrégats superparamagnétiques encapsulés, ledit procédé comprenant les étapes suivantes :
a) revêtir des nanoparticules superparamagnétiques avec une couche hydrophobe,
b) disperser lesdites nanoparticules superparamagnétiques dans un solvant organique apolaire,
c) évaporer ledit solvant organique apolaire sous vide, de manière à obtenir une cire constituée de nanoparticules superparamagnétiques revêtues d'une couche hydrophobe,
d) émulsionner ladite cire avec un premier tensioactif cationique dispersé dans une solution aqueuse,
e) effectuer une première homogénéisation de ladite solution,
f) ajouter un composé basique et un solvant organique polaire dans ladite solution,
g) effectuer une deuxième homogénéisation de ladite solution,
h) chauffer ladite solution à une température d'au moins 60°C,
i) ajouter un précurseur de silicate dans ladite solution,
j) agiter ladite solution à une température d'au moins 60°C, et
k) effectuer une centrifugation pour donner des nanoparticules de silice mésoporeuses avec des agrégats superparamagnétiques encapsulés ;
**caractérisé en ce que**
un second tensioactif cationique est ajouté entre lesdites étapes (j) et (k).

2. Procédé selon la revendication 1, **caractérisé en ce que** lesdites nanoparticules superparamagnétiques sont des nanoparticules superparamagnétiques d'oxyde métallique, de préférence Fe₃O₄, CoFe₂O₄, MnFe₂O₄, ou Zn_{0.4}Co_{0.6}Fe₂O₄.

3. Procédé selon la revendication 1 ou 2, **caractérisé en ce que** ledit premier tensioactif cationique et/ou ledit second tensioactif cationique est un tensioactif à base d'ammonium quaternaire, de préférence étant sélectionné dans le groupe comprenant le chlorure de cétyltriméthylammonium, le bromure de cétrimonium, ou le 1,2-dioléoyle-3-triméthylammonium-propane.

4. Procédé selon l'une quelconque des revendications 1 à 3, **caractérisé en ce que** le nombre d'équivalent de la quantité totale desdits premier et second tensioactifs cationiques est compris entre 15 et 25 équivalents desdites nanoparticules superparamagnétiques, de préférence étant égal à 18.

5. Procédé selon l'une quelconque des revendications 1 à 4, **caractérisé en ce que** le premier tensioactif cationique est compris entre 5% et 15% de la quantité totale desdits premier et second tensioactifs cationiques, ledit premier tensioactif cationique étant de préférence équivalent à 10% de la quantité totale desdits premier et second tensioactifs cationiques.

6. Méthode de fabrication d'une bicouche lipidique supportée chargée négativement entourant des nanoparticules de silices mésoporeuses avec des agrégats superparamagnétiques encapsulés, ledit procédé comprenant le procédé de fabrication de nanoparticules de silice mésoporeuses avec des agrégats superparamagnétiques encapsulés selon l'une quelconque des revendications 1 à 5 et les étapes suivantes consistant à :
a) ajouter du (3-aminopropyl)triéthoxysilane, alias APTES,
b) effectuer une purification,
c) ajouter une formulation de lipides, lesdits lipides étant la 1,2-dioléoyl-sn-glycéro-3-phospho-L-sérine, alias DOPS, le cholestérol et au moins un lipide différent de DOPS et du cholestérol,
d) effectuer une ultrasonication, et
e) effectuer une centrifugation.

7. Méthode selon la revendication 6, dans laquelle le nombre d'équivalent de cholestérol par rapport à un équivalent de DOPS est compris entre 2,30 et 2,70, de préférence entre 2,40 et 2,60, plus préférentiellement le nombre d'équivalent de cholestérol par rapport à un équivalent de DOPS étant de 2,50.

8. Procédé de fabrication d'une couche d'alginate sur une bicouche lipidique supportée chargée négativement entourant des nanoparticules de silice mésoporeuses avec des agrégats superparamagnétiques encapsulés, ledit procédé comprenant le procédé de fabrication d'une bicouche lipidique supportée chargée négativement entourant des nanoparticules de silice mésoporeuses avec des agrégats superparamagnétiques encapsulés selon à l'une quelconque des revendications 6 et 7, et les étapes suivantes :
a) fournir une bicouche lipidique supportée chargée négativement entourant une nanoparticule de silice mésoporeuse avec des agrégats superparamagnétiques encapsulés, dans laquelle ladite bicouche lipidique supportée chargée négativement comprend au moins un phospholipide,
b) ajouter une formulation de lipides, lesdits lipides étant préférentiellement le 1,2-dioléoyle-3-triméthylammonium-propane, alias DOTAP, le cholestérol et au moins un lipide différent de DOTAP et du cholestérol,
c) effectuer une ultra-sonication,
d) ajouter une solution aqueuse d'alginate de sodium, et
e) réticuler ledit alginate de sodium.

9. Procédé selon la revendication 8, **caractérisé en ce que** ledit au moins un lipide différent de DOTAP et du cholestérol est la 1,2-dipalmitoyle-sn-glycéro-3-phosphocholine, alias DPPC.

10. Nanoparticule de silice mésoporeuse comprenant au moins un agrégat superparamagnétique encapsulé, **caractérisée en ce que** ledit au moins un agrégat superparamagnétique encapsulé est centré à l'intérieur de ladite nanoparticule de silice mésoporeuse, et ladite nanoparticule de silice mésoporeuse comprenant trois ou cinq agrégats superparamagnétiques encapsulés alignés.

11. Nanoparticule de silice mésoporeuse selon la revendication 10, **caractérisée en ce que** ledit agrégat superparamagnétique encapsulé est un agrégat superparamagnétique d'oxyde métallique, de préférence Fe₃O₄, CoFe₂O₄, MnFe₂O₄, ou Zn_{0.4}Co_{0.6}Fe₂O₄.

12. Nanoparticule de silice mésoporeuse selon la revendication 10 ou 11, **caractérisée en ce que** ladite nanoparticule mésoporeuse comprend une bicouche lipidique supportée chargée négativement et comprend en outre une couche externe d'alginate.

13. Nanoparticule de silice mésoporeuse selon la revendication 12, destinée à être utilisée dans un traitement d'hyperthermie magnétique ou dans un après-traitement d'une tumeur cancéreuse.

14. Nanoparticule de silice mésoporeuse selon la revendication 12, destinée à être utilisée dans dans une administration loco-régionale et/ou topique en tant qu'agent cosmétique et/ou agent dermatologique.

15. Nanoparticule de silice mésoporeuse selon l'une quelconque des revendications 10 à 14, destinée à être utilisée dans l'imagerie par résonance magnétique ou destinée à être utilisée simultanément dans l'imagerie par résonance magnétique et l'imagerie de fluorescence.
